(19) **Europäisches Patentamt European Patent Office Office européen des brevets**

(11) **EP 1 233 700 B1**

(12) **FASCICULE DE BREVET EUROPEEN**

(45) Date de publication et mention
de la délivrance du brevet:
**21.02.2007 Bulletin 2007/08**

(51) Int Cl.:
***A61B 6/00*** (2006.01)

(21) Numéro de dépôt: **00985380.5**

(86) Numéro de dépôt international:
**PCT/FR2000/003358**

(22) Date de dépôt: **01.12.2000**

(87) Numéro de publication internationale:
**WO 2001/039666 (07.06.2001 Gazette 2001/23)**

(54) **PROCEDE D'UTILISATION D'UN SYSTEME D'OSTEODENSITOMETRIE, PAR RAYONNEMENT X BI-ENERGIE**

VERFAHREN ZUR ANWENDUNG EINES KNOCHENDICHTEMESSUNGSSYSTEMS MITTELS RÖNTGENSTRAHLUNG BEI ZWEI ENERGIEN

METHOD FOR USING A BONE DENSITOMETRY SYSTEM, WITH DUAL-ENERGY X-RADIATION

(84) Etats contractants désignés:
**DE FR GB IT**

(30) Priorité: **03.12.1999 FR 9915275**

(43) Date de publication de la demande:
**28.08.2002 Bulletin 2002/35**

(73) Titulaire: **COMMISSARIAT A L'ENERGIE ATOMIQUE**
**75015 Paris (FR)**

(72) Inventeurs:
• **DINTEN, Jean-Marc**
**F-69008 Lyon (FR)**
• **ROBERT-COUTANT, Christine**
**F-38410 Saint-Martin D'Uriage (FR)**

(74) Mandataire: **Poulin, Gérard et al**
**Société BREVATOME**
**3, rue du Docteur Lancereaux**
**75008 Paris (FR)**

(56) Documents cités:
**WO-A1-90/10859      US-A- 5 150 394**
**US-A- 5 457 724      US-A- 5 745 544**
**US-A- 5 841 832**

**Description**

**Domaine technique**

La présente invention concerne un procédé d'utilisation d'un système d'ostéodensitométrie par rayons X.

On rappelle que l'ostéodensitométrie par rayons X est une technique de mesure de masses et de densités osseuses à partir d'acquisitions radiographiques effectuées à une pluralité d'énergies.

On utilise en général deux énergies que l'on appelle respectivement "haute énergie" et "basse énergie".

Diverses configurations d'une chaîne de mesure (source et détecteur de rayons X) sont envisageables pour effectuer les mesures.

On distingue trois familles de systèmes selon les types de rayonnements utilisés :

- les systèmes à pinceau de rayonnement ("pencil beam systems") qui utilisent une source de rayons X collimatée par un trou et un monodétecteur de rayons X qui est également collimaté,
- les systèmes à faisceau en éventail ("fan beam systems") qui utilisent une source de rayons X collimatée par une fente et un détecteur linéaire de rayons X, et
- les systèmes à faisceau conique ("cone beam systems") qui utilisent un détecteur bidimensionnel de rayons X.

L'invention concerne plus particulièrement la mise en oeuvre de systèmes d'ostéodensitométrie par rayonnement X bi-énergie à faisceau conique.

**Etat de la technique antérieure**

Les principes méthodologiques de l'ostéodensitométrie par rayonnement X bi-énergie et les principales solutions techniques actuellement utilisées sont connus par les deux documents suivants auxquels on se reportera :

[1] "Technical Principles of Dual Energy X-Ray Absorptiometry", G.M. Blake et I. Fogelman, Seminars in Nuclear Medicine, Vol XXVII, n°3, juillet 1997, pages 210 à 228 et

[2] "The Evaluation of Osteoporosis : Dual Energy X-Ray Absorptiometry and Ultrasound in Clinical Practice", Second Edition, G.M. Blake, H.W. Wahner et I. Fogelman, Martin Dunitz Editor, 1999, ISBN 1-85317-472-6.

On se reportera plus particulièrement aux chapitres 3, 4 et 5 du document [2] où sont décrits les principes de mesure de densités osseuses à deux énergies et les systèmes connus pour faire de telles mesures.

Pour réaliser le diagnostic et le suivi thérapeutique de l'ostéoporose en des sites pertinents (colonne vertébrale, hanches, avant-bras, corps entier), les systèmes commercialement disponibles sont de type "pencil beam" et "fan beam". Ces systèmes utilisent des collimateurs qui limitent les rayonnements parasites, en particulier les rayonnements diffusés ("scattered"). Par ailleurs, la géométrie de ces systèmes d'acquisition limite les déformations géométriques liées à la dimension du détecteur, qui résulteraient, en particulier, de la conicité du faisceau de rayons X.

De plus, ces systèmes commercialement disponibles utilisent un balayage en effectuant successivement, pour chaque position de la chaîne d'acquisition, une mesure à haute énergie et une mesure à basse énergie. Cela assure une parfaite cohérence entre les zones du patient qui sont observées lors des deux mesures.

Les systèmes à faisceau conique commercialement disponibles ne sont prévus que pour faire des mesures de densité osseuse sur des zones périphériques telles que les phalanges, les mains, les avant-bras et les calcanéums. Par rapport aux deux autres familles de systèmes, la mise en oeuvre de ces systèmes à faisceau conique est plus délicate.

En effet, le rayonnement diffusé engendré par l'interaction entre le rayonnement X incident et la partie étudiée du corps est important et doit être traité. De plus, la conicité rend la mesure dépendante de la position de la zone anatomique considérée dans le champ image. En outre, durant le temps de lecture du capteur bi-dimensionnel, le patient peut avoir bougé entre la mesure à haute énergie et la mesure à basse énergie.

Si les systèmes du genre "cone beam" commercialement disponibles sont limités à l'analyse d'extrémités du corps humain telles que les mains, les avant-bras et les calcanéums, c'est parce que ces zones sont peu épaisses et ont de faibles dimensions. En conséquence, les rayonnements parasites sont limités et le positionnement peut être consolidé, par exemple au moyen d'un système de fixation pour un avant-bras ou un calcanéum.

En revanche, la mise en oeuvre de tels systèmes à faisceau conique pour des zones anatomiques de dimensions plus importantes, en particulier la colonne vertébrale ou les hanches, zones essentiellement utilisées pour le diagnostic et le suivi de l'ostéoporose, nécessite de prendre en compte les phénomènes parasites ainsi que l'importante conicité du rayonnement X, et de s'adapter aux mouvements potentiels du patient.

On connaît déjà des systèmes d'ostéodensitométrie de zones bidimensionnelles, comprenant une source apte à fournir des flux de rayons X à au moins deux énergies, un écran-convertisseur apte à transformer les rayons X en photons de lumière visible, un système optique de reprise d'image et une caméra CCD, par les documents suivants auxquels on se reportera :

[3] brevet US-5,150,394 du 22 septembre 1992, "Dual-Energy System for Quantitative Radiographic Imaging", (Andrew Karellas), et
[4] Demande internationale publiée le 14 novembre 1996, n° de publication WO 96/35372, "A System for Quantitative Radiographic Imaging", (Andrew Karellas).

**Exposé de l'invention**

La présente invention a pour objet un procédé d'utilisation d'un système d'ostéodensitométrie du genre de ceux qui sont décrits dans les documents [3] et [4], ce procédé permettant d'augmenter la précision et la reproductibilité des mesures de densité osseuse dans des zones anatomiques d'un patient, à partir de radiographies bidimensionnelles de ces zones anatomiques, acquises à une pluralité d'énergies.
A cet effet, l'invention prend en compte le mouvement du patient entre deux mesures, prises à des énergies différentes.
Pour ce faire, la présente invention utilise une mise en correspondance ("matching") entre une image acquise à haute énergie et une image acquise à basse énergie et en outre, de préférence, un moyen d'aide au positionnement du patient.
De façon précise, la présente invention a pour objet un procédé d'utilisation d'un système d'ostéodensitométrie par rayons X à au moins deux énergies, à faisceau conique, ce système comprenant une source de rayons X apte à fournir un faisceau conique de rayons X à au moins une première énergie, appelée haute énergie, et une deuxième énergie, appelée basse énergie et inférieure à la première énergie, un détecteur bidimensionnel de rayons X et des moyens électroniques de traitement des images fournies par ce détecteur, ce procédé étant caractérisé en ce qu'on acquiert l'image à basse énergie d'une zone anatomique d'un patient, et pour prendre en compte les mouvements du patient dans les mesures de densité osseuse, on acquiert l'image à haute énergie de cette zone anatomique et l'on met en correspondance ces images respectivement acquises à basse énergie et à haute énergie, avant de construire la carte des densités osseuses de la zone anatomique.
Selon un mode de mise en oeuvre particulier du procédé objet de l'invention, pour mettre en correspondance les images respectivement acquises à haute et basse énergies :

-    on extrait respectivement de ces images les ensembles de contours des zones osseuses de la zone anatomique,

-    on recherche la transformation plane optimale permettant de mettre l'ensemble de contours relatif à l'image acquise à haute énergie en correspondance avec l'ensemble de contours relatif à l'image acquise à basse énergie (ou inversement, selon une variante),

-    on utilise cette transformation pour amener l'image acquise à haute énergie dans le référentiel de l'image acquise à basse énergie (ou inversement, selon la variante ci-dessus), et

-    on combine ces deux images pour déterminer la carte de mesure des densités osseuses.

Selon un mode de mise en oeuvre préféré du procédé objet de l'invention, avant de faire les acquisitions à haute et basse énergies, on fait un cliché de radioscopie, c'est-à-dire un cliché à faible dose de rayons X, de la zone anatomique du patient pour aider à positionner cette zone dans le système.
De préférence, lorsque le patient est soumis à un premier examen, on utilise ce cliché à faible dose de rayons X pour rétroagir sur la mécanique du système afin de positionner la zone anatomique par rapport à un référentiel préétabli.
De préférence, lorsque le patient est soumis à un examen de suivi, on utilise le cliché à faible dose de rayons X pour placer la zone anatomique dans une position identique à celle qu'elle occupait lors de l'examen précédent.
De façon plus explicite, dans le mode de réalisation préféré, avant de faire les acquisitions à haute et basse énergies, on fait un cliché, à faible dose de rayons X et à une seule énergie, de la zone anatomique du patient. A partir d'une détection des contours osseux sur ce cliché, on déduit les paramètres géométriques permettant soit de centrer la zone anatomique dans le champ d'acquisition en cas de premier examen, soit de positionner la zone anatomique par rapport au champ d'acquisition de la même façon que dans l'examen précédent si ce n'est pas le premier examen. Dans les deux cas, ce positionnement est effectué par déplacement du patient par rapport au système source-détecteur, ou du système source-détecteur par rapport au patient, par commande manuelle ou automatique.
Selon un mode de réalisation particulier de l'invention, avant de faire les acquisitions à haute et basse énergies, on fait un cliché à faible dose de rayons X, pour adapter la dose d'irradiation par réglage du flux de rayons X en modifiant le courant appliqué à la source de rayons X utilisée et/ou la tension appliquée à cette source.

Selon un autre mode de réalisation particulier, avant de faire les acquisitions à haute et basse énergie, on fait un cliché à faible dose de rayons X pour positionner automatiquement des caches permettant de limiter la zone d'irradiation.

On connaît déjà la possibilité d'utiliser un cliché préalable à l'examen (également appelé « prescan scout data ») pour « centrer » le patient dans le domaine de la tomographie par le brevet US 5457724 « Automatic field of view and patient centering determination from prescan scout data » du 10 octobre 1995.

Dans ce brevet, avant de reconstruire une coupe tomographique d'un patient, on acquiert deux projections monodimensionnelles (éventail ou « fan-beam ») à 0° et 90° de cette coupe. On détecte les points correspondant aux bords du patient dans les deux projections, et on en déduit la position du centre de la zone et la taille d'acquisition tomographique. Ces paramètres sont donnés à l'opérateur et peuvent être utilisés pour déplacer le patient afin de mieux le centrer pour l'acquisition tomographique. Le but est d'obtenir la meilleure qualité d'image possible, les systèmes tomographiques étant étudiés pour que l'atténuation maximale se trouve au centre de la zone d'acquisition, et les corrections de durcissement de spectre étant effectuées en fonction de la taille du champ d'acquisition.

Dans la présente invention il s'agit de radiologie (avec un détecteur bidimensionnel) et non pas de tomographie (avec un détecteur « fan-beam » en rotation). L'image finale est une projection bidimensionnelle et pas une coupe reconstruite. De plus, dans le mode de réalisation préféré on utilise un seul cliché préalable et non pas deux clichés à 90° l'un de l'autre. En outre, un but de l'invention est la reproductibilité de la mesure de masse osseuse calculée à partir de l'image, et pas la qualité de l'image elle-même. De plus, dans les systèmes tomographiques le recentrage du patient n'est pas automatique (il l'est en hauteur mais pas en largeur, car le déplacement latéral de la table n'est pas prévu ou n'est pas nécessaire).

La « reproductibilité » est la propriété de l'appareil de mesure de donner la même mesure pour différents examens sur le même patient (supposé de densité osseuse constante) et le même site anatomique. Dans le cas d'un patient dont la masse osseuse varie dans le temps, sous l'effet d'une maladie ou d'un traitement par exemple, cette propriété de reproductibilité permet de quantifier ces variations de la masse osseuse.

De plus, dans la présente invention on peut utiliser ce « prescan scout data » pour positionner automatiquement des caches permettant de limiter la zone d'irradiation à la zone osseuse, ce qui n'est pas possible en tomographie sous peine de projections tronquées. De cette façon la dose d'irradiation reçue par le patient est minimisée.

Rappelons que la mise en correspondance d'images (« image matching ») est utilisée dans plusieurs domaines du traitement d'images tels que, par exemple, la vision stéréoscopique, l'analyse de séquences d'images et la fusion d'images de modalités ou de natures différentes.

Différentes approches ont été développées, par exemple la mise en correspondance entre les nappes de niveaux de gris ou l'extraction de primitives et la mise en correspondance entre les primitives.

A ce sujet, on se reportera au document suivant :

[5] "Overview of Image Matching Techniques", C. Heipke, Proceedings of OEEEPE Workshop on the Application of Digital Photogrammetric Workstation, Kölb O. Editor, OEEEPE Official publications, n°33, pages 173 à 189, 1996.

En ce qui concerne le positionnement du patient, sur les systèmes de type "pencil beam" ou "fan beam", un premier positionnement du patient s'effectue à l'aide d'un pointeur laser qui repère la zone d'examen à partir d'observations morphologiques externes. Ensuite, le balayage débute. Si le patient est bien positionné l'examen se poursuit mais si à l'observation sur l'écran des premières lignes acquises le positionnement n'est pas bon, l'opérateur arrête tout et effectue un nouveau positionnement puis relance l'examen.

A ce sujet, on se reportera au document [2] pages 198 à 200 pour ce qui concerne la colonne vertébrale et aux pages 265 à 267 pour ce qui concerne la hanche.

Une étude récente a montré que, pour des systèmes du genre "pencil beam", le repositionnement avait lieu dans 50% des cas et que, pour environ 10% des examens, il fallait repositionner jusqu'à trois fois le patient. A ce sujet, on se reportera au document suivant :

[6] Insights, vol. 10, n°1, mars 1999, pages 10 et 11 (revue éditée par la Société Hologic), "Independent survey reveals surprising, disappointing results for Lunar users".

Sur les systèmes du genre "cone beam" connus, utilisés pour des examens de zones périphériques, le positionnement du patient est assuré par un système mécanique d'aide au positionnement, par exemple une poignée pour l'avant-bras et une cuvette formée pour le talon.

**Brève description des dessins**

La présente invention sera mieux comprise à la lecture de la description d'exemples de réalisation donnés ci-après, à titre purement indicatif et nullement limitatif, en faisant référence aux dessins annexés sur lesquels :

- la figure 1 est une vue schématique d'un système d'ostéodensitométrie osseuse par rayonnement X à deux énergies, à faisceau conique, qui est utilisable pour la mise en oeuvre de l'invention,

- les figures 2 et 3 sont des organigrammes de procédures qui sont utilisées dans des modes de mise en oeuvre particuliers de l'invention,

- la figure 4 représente le diagramme fonctionnel d'un mode de réalisation avantageux d'un procédé de correction des défauts d'une image, qui est utilisable dans la présente invention, et

- la figure 5 représente le diagramme fonctionnel d'une variante de ce mode de réalisation avantageux du procédé de correction des défauts d'une image, qui est également utilisable dans la présente invention.

**Exposé détaillé de modes de réalisation particuliers**

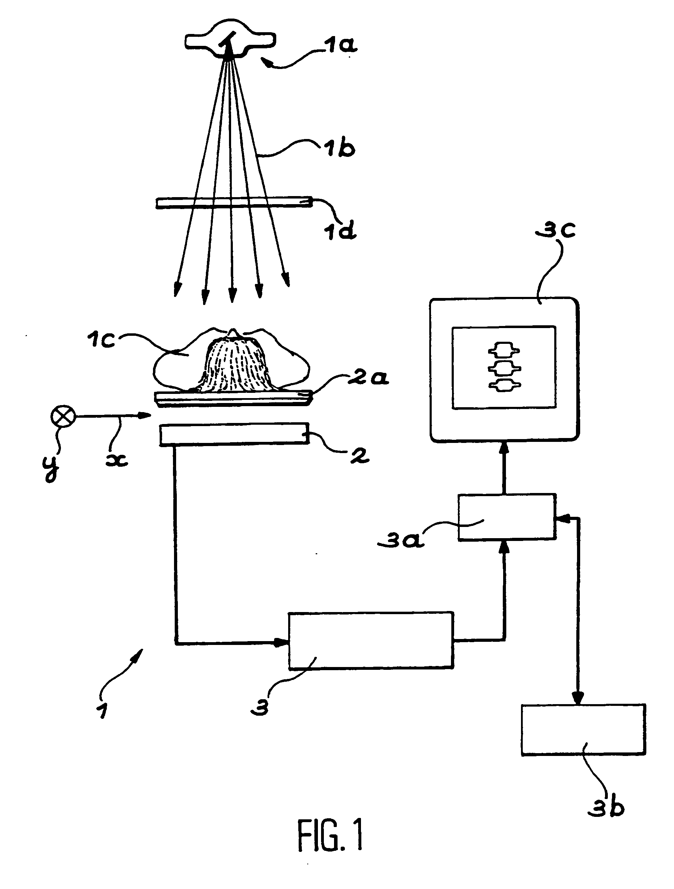

On voit sur la figure 1 un système d'ostéodensitométrie osseuse 1 qui comprend une source de rayons X la apte à envoyer un faisceau conique 1b de rayons X vers le corps d'un patient 1c en cours d'examen. Cette source 1a est apte à émettre des rayonnements X correspondant respectivement à deux niveaux distincts d'énergie. Ces deux niveaux sont utilisés pour obtenir deux images distinctes du patient.

Un filtre amovible 1d est interposable entre la source la et le patient 1c et sert à améliorer les qualités spectrales du faisceau.

Le système 1 comprend aussi un détecteur bidimensionnel 2 qui est très schématiquement représenté en coupe transversale sur la figure 1 et destiné à détecter les rayons X émis par la source et ayant traversé le patient 1c. ce détecteur 2 est parallèle à un plan défini par deux directions orthogonales x et y.

Ce patient est placé sur un support approprié 2a, par exemple un lit, qui est transparent aux rayons X. Dans l'exemple de la figure 1 la source 1a (munie de l'éventuel filtre 1d) est placée au-dessus du patient reposant sur le support tandis que le détecteur est placé en-dessous de ce support.

Des moyens non représentés sont prévus pour déplacer le support 2a par rapport à la source 1a et au détecteur 2, qui sont alors fixes, ou sont prévus pour déplacer la source 1a et le détecteur 2 par rapport au support 2a qui est alors fixe, ces déplacements ayant lieu parallèlement aux directions x et y.

Dans l'invention on peut utiliser tout type de détecteur bidimensionnel, par exemple un écran sensible aux rayons X et apte à fournir directement un signal électronique représentatif de l'image acquise par le détecteur sous forme de pixels.

Au lieu de cela on peut utiliser un écran-scintillateur prévu pour recevoir les rayons X ayant traversé le patient et pour convertir ces rayons X en lumière visible. Cette dernière est alors envoyée, par l'intermédiaire d'un miroir, à un capteur CCD muni d'un objectif et comprenant un réseau de pixels photosensibles.

Sur la figure 1, on voit aussi un dispositif 3 du genre contrôleur de CCD ou analogue qui lit, pixel par pixel, la représentation d'image fournie par le détecteur et qui numérise cette représentation. La représentation ainsi numérisée est stockée dans une mémoire 3a.

Un ordinateur 3b est prévu pour traiter les images ainsi mémorisées.

Un dispositif d'affichage 3c, comprenant par exemple un tube à rayons cathodiques, est prévu pour afficher les images avant ou après ce traitement.

Un tel système est utilisable pour mettre en oeuvre un procédé conforme à l'invention, selon lequel, en vue d'obtenir une bonne précision et une bonne reproductibilité de la mesure de densité osseuse,

- on réalise une mise en correspondance entre les images respectivement acquises à haute énergie et à basse énergie et

- de préférence, on utilise en outre un premier cliché, à faible dose, de type scopie, pour aider à positionner le patient dans le système d'ostéodensitométrie.

Considérons d'abord la mise en correspondance entre l'acquisition à haute énergie et l'acquisition à basse énergie.

Afin de pouvoir construire une carte de densité de l'os, il est indispensable que, pour chacune de ces acquisitions, les rayons X aient eu le même parcours dans la zone anatomique.

Pour un système du genre "cone beam", le patient est susceptible de bouger entre l'acquisition à haute énergie et l'acquisition à basse énergie. Une façon d'éviter cela est de réaliser ces deux acquisitions dans un court intervalle de temps, inférieur à 200 ms, comme cela se fait sur les systèmes du genre "pencil beam" et "fan beam". Mais, à la différence de ces systèmes, pour le système du genre "cone beam", il faut traiter des images bidimensionnelles c'est-à-dire des images comportant une grande quantité de données, nécessitant un temps de lecture important. Cela nécessite des technologies très sophistiquées et très onéreuses.

L'invention permet de ne pas être soumis à cette contrainte de rapidité entre les deux acquisitions, en traitant a posteriori les acquisitions à haute et basse énergies.

Si les deux acquisitions sont réalisées en quelques secondes, par exemple environ 10 secondes, la zone anatomique est susceptible d'avoir subi un léger déplacement. En utilisant les acquisitions à haute et basse énergies telles quelles, on induit une erreur dans la mesure de densité osseuse. Pour remédier à cet inconvénient, on propose, dans l'invention, de prendre en compte les mouvements de la zone anatomique entre les deux acquisitions et, pour ce faire, on propose de mettre en correspondance les acquisitions à haute et basse énergies avant de construire la carte des densités osseuses.

Considérons ensuite l'utilisation d'un cliché de scopie pour le positionnement du patient.

Etant donné que, pour un système du genre "cone beam", on dispose d'un capteur bidimensionnel qui permet, en une seule acquisition, d'avoir une vision globale de la zone analysée, on propose de réaliser un cliché à faible dose (cliché de scopie) avant les acquisitions à haute et basse énergies pour aider au positionnement du patient.

Si ce patient est soumis à son premier examen, on utilise ce cliché de scopie pour rétroagir sur la mécanique du système (c'est-à-dire commander la mécanique du dispositif d'acquisition d'images de façon qu'il se positionne correctement par rapport au patient ou inversement) afin de positionner la zone anatomique par rapport à un référentiel préétabli.

Si l'examen est un examen de suivi du patient, on utilise le cliché de scopie pour mettre la zone anatomique dans une position identique à celle qu'elle occupait lors du précédent examen (lors duquel on avait fait également un cliché de scopie).

Ce type de démarche permet d'augmenter sensiblement la reproductibilité des mesures effectuées avec des systèmes de type "cone beam".

En raison de la conicité du faisceau, la mesure dépend de la position de la zone anatomique dans ce faisceau.

En utilisant le cliché de scopie pour positionner de façon identique la zone anatomique du patient par rapport à un référentiel donné ou pour assurer la cohérence entre deux examens, on obtient une bonne reproductibilité de l'examen.

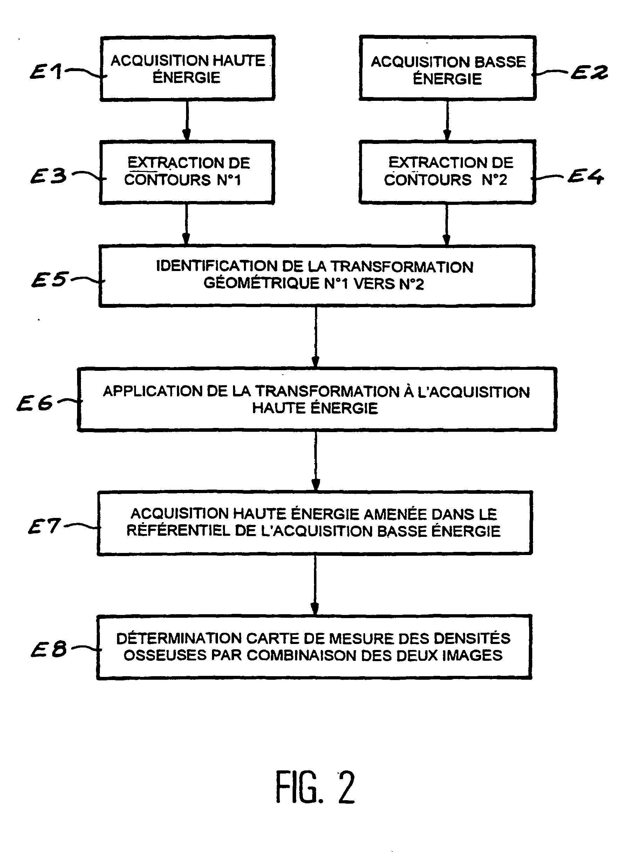

A titre d'exemple, pour la mise en correspondance entre les acquisitions à haute et basse énergies, on procède de la façon suivante, illustrée par l'organigramme de la figure 2 :

   1.1. on effectue ces acquisitions à haute et basse énergies (étapes E1 et E2) ;
   1.2. on extrait respectivement de ces deux acquisitions les deux ensembles (n°1 et n°2) de contours des zones osseuses (étapes E3 et E4) ;
   1.3. on cherche la meilleure (au sens des moindres carrés par exemple) transformation géométrique plane (composition d'homothétie, translation et rotation) permettant de mettre en correspondance les deux ensembles de contours (étape E5) ;
   1.4. on utilise cette transformation pour amener l'image acquise à haute énergie dans le référentiel de l'image acquise à basse énergie (étapes E6 et E7) ;
   1.5. on combine ces deux images pour déterminer la carte de mesure des densités osseuses (étape E8).

En ce qui concerne l'utilisation d'un cliché de radioscopie (en abrégé scopie) pour le positionnement, un exemple de mise en oeuvre pour un patient soumis à son premier examen consiste à :

   2.1. réaliser un cliché à faible dose ;
   2.2. extraire de cette acquisition les contours des zones osseuses ;
   2.3. repérer des points caractéristiques dans la carte de contour, par exemple identification de vertèbres ou repérage de points caractéristiques sur le col du fémur ;
   2.4. construire la fonction de type translation qui permet de placer au mieux ces points par rapport à une position standard définie au préalable ;
   2.5. rétroagir sur la mécanique de positionnement pour se ramener à la position standard ;
   2.6. réaliser les acquisitions à haute et basse énergies.

Dans le cas d'un patient soumis à un examen de suivi thérapeutique, l'étape de construction de fonction 2.4 est remplacée par les deux étapes suivantes :

2.4.1 récupérer les positions des points caractéristiques dans les acquisitions d'un examen précédent du patient ;
2.4.2 construire la fonction de type translation qui permet de mettre au mieux eh correspondance les points caractéristiques du cliché de scopie par rapport à leur position dans un examen antérieur.

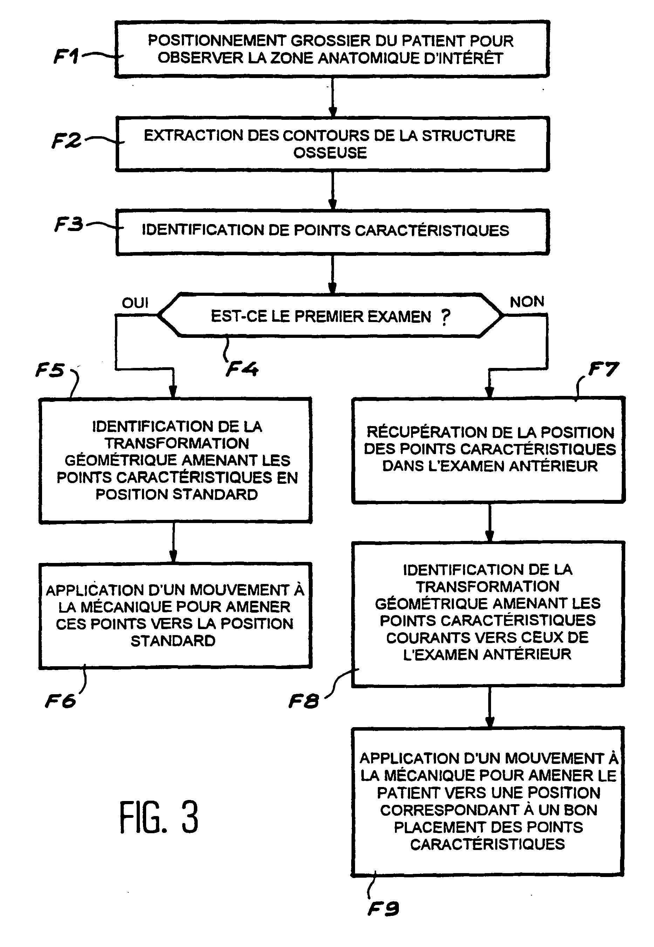

Tout ceci est précisé par l'organigramme de la figure 3 :

- Étape F1 : on positionne grossièrement le patient pour observer la zone anatomique d'intérêt (voir 2.1)
- Étape F2 : on extrait les contours de la structure osseuse (voir 2.2)
- Étape F3 : on identifie les points caractéristiques (voir 2.3)
- Étape F4 : on se demande s'il s'agit du premier examen
- Si oui on va à l'étape F5 dans laquelle on identifie la transformation géométrique amenant les points caractéristiques en position standard (voir 2.4) puis on va à l'étape F6 dans laquelle on applique un mouvement à la mécanique pour amener ces points vers la position standard (voir 2.5)
- Si non on va à l'étape F7 dans laquelle on récupère la position des points caractéristiques dans l'examen antérieur (voir 2.4.1) puis on va à l'étape F8 dans laquelle on identifie la transformation géométrique amenant les points caractéristiques courants vers ceux de l'examen antérieur (voir 2.4.2) puis on va à l'étape F9 dans laquelle on applique un mouvement à la mécanique pour amener le patient vers une position correspondant à un bon placement des points caractéristiques (voir 2.5).

De plus, dans la présente invention, on peut utiliser le cliché de radioscopie pour

1/ adapter la dose d'irradiation par réglage du flux de rayons X en modifiant le courant appliqué à la source de rayons X utilisée et/ou la tension appliquée à cette source
2/ positionner automatiquement des caches permettant de limiter la zone d'irradiation, ce qui n'est pas possible en tomographie sous peine de projections tronquées.

De cette façon la dose d'irradiation reçue par le patient est minimisée.
On donne dans ce qui suit des précisions sur le traitement des images selon un mode de réalisation préféré du procédé objet de l'invention.
Les étapes a) à e) qui suivent constituent des étapes préparatoires « hors ligne ».

a) La caractérisation du décalage ("offset") en chacun des pixels qui composent une image est réalisée par acquisition d'images de noir (images sans rayonnement X), notées NOIR, et la caractérisation en gain s'obtient par l'acquisition d'images, à plein flux à chaque énergie de fonctionnement c'est-à-dire avec le flux maximum de rayonnement X, sans objet ou avec un objet d'épaisseur uniforme, notées PFL. Afin d'obtenir des caractéristiques peu affectées par le bruit d'acquisition, on réalise un nombre important d'acquisitions dont on fait la moyenne.
b) La carte des pixels en défaut est obtenue par l'analyse d'une acquisition à plein flux ou d'un objet d'épaisseur uniforme.
c) La caractérisation des distorsions géométriques est obtenue par l'acquisition d'images de grilles avec des motifs répartis régulièrement ; à partir de la description géométrique de ces grilles et de leurs images on construit la fonction de distorsion géométrique du système. Afin de séparer cet effet de celui de la conicité, il faut placer les grilles au contact du détecteur (référence 2 dans l'exemple de la figure 1).
d) Le calibrage géométrique du système est réalisé par l'acquisition d'une grille de géométrie connue que l'on place à des positions parfaitement identifiées par rapport au détecteur. Cela permet de remonter à une position de la source de rayons X.
e) L'étalonnage "en densité" est le même pour tous les pixels de détection (pixels obtenus avec le détecteur 2 dans l'exemple de la figure 1), les différences de gain ayant été corrigées préalablement. On utilise un fantôme composé d'un certain nombre d'éléments, chaque élément étant composé d'un mélange de deux matériaux de base, d'épaisseurs connues. Les matériaux de base peuvent être un matériau n°1 simulant l'os et un matériau n°2 simulant les tissus mous. On s'assure mécaniquement que les mesures à haute et basse énergie sont acquises perpendiculairement à chaque élément, sans avoir à effectuer de correction de conicité. Un dispositif de collimation (non représenté sur la figure 1), que l'on dispose par exemple entre la source de rayonnement et le fantôme ou entre le fantôme et le détecteur, garantit que ces mesures sont exemptes de rayonnement diffusé. A partir de ces mesures d'étalonnage, on estime les fonctions qui transforment un couple (mesure à basse énergie, mesure à haute énergie) en un couple (épaisseur équivalente de matériau de base n°1, épaisseur équivalente du matériau de base n°2).

À ces étapes préparatoires succèdent des étapes de traitement de l'image. On calcule une image corrigée par la formule suivante :

## image corrigée=(Acquis-NOIR)/(PFL-NOIR)

où "Acquis" représente successivement l'acquisition à basse énergie et l'acquisition à haute énergie.

Et les pixels en défaut isolés sont corrigés par un calcul de moyenne locale ou par un calcul de médiane. Pour les paquets de pixels on utilise une approche fondée sur un procédé pour corriger les défauts d'une image provenant d'un détecteur de rayons X (ou γ) de type matriciel (définition d'un degré de confiance aux différents pixels et mise en oeuvre d'une convolution normalisée), ce procédé (invention de R. Guillemaud - demande de brevet français n°EN 98 15044 du 30 novembre 1998) étant expliqué à la fin de la présente description.

A partir de la fonction de distorsion identifiée dans l'une des étapes préparatoires "hors ligne", c'est-à-dire l'étape c), on corrige la position des pixels observés et, par interpolation, on reforme une image échantillonnée sur une grille à pas ("pitch") régulier.

La mise en correspondance entre l'acquisition à basse énergie et l'acquisition à haute énergie est effectuée par extraction d'un ensemble de contours dans chacune des deux acquisitions (contours liés à l'interface tissus/os) et par la détermination des paramètres de rotation et de translation permettant de passer d'un ensemble de contours à l'autre.

Pour le rayonnement diffusé, on utilise le procédé de correction du flux diffusé dans des images de radiographie numérique qui est décrit dans la demande internationale publiée le 20 août 1998, n° de publication WO 98/36380 (M. Darboux et J.M. Dinten), et qui utilise un modèle tridimensionnel des zones anatomiques, en décrivant celles-ci comme des zones composées de deux matériaux : les tissus mous (valeur correspondant à une proportion de graisse et de tissus maigre chez un individu standard) et les os. Les dimensions de ce modèle tridimensionnel, qui sont parallèles au plan de projection, sont données par l'analyse des projections. Les cotes en profondeur résultent d'une estimation de l'épaisseur de la zone anatomique (dans une zone sans os) et de données a priori fournies par les traités d'anatomies (par exemple, position de la colonne vertébrale par rapport au dos pour une examen en posterio-anterior). Ce procédé connu consiste ensuite en une approximation des équations de Klein et Nishina en utilisant cette description a priori et conduit à l'expression du rayonnement diffusé comme une fonction non linéaire du rayonnement direct. Pour plus de détails, on se reportera à cette demande internationale WO 98/36380.

Pour la détermination de l'image d'os, on applique les fonctions qui ont été calculées lors de l'étape "hors ligne" afin de calculer une image "os" et une image "tissus mous". Les tissus mous, avec une proportion variable de graisse, apparaissent sur les deux images à la fois et biaisent l'image d'os. Une segmentation permet de déterminer les zones ne contenant pas d'os ; sur ces zones on peut calculer une "ligne de base" qui correspond à la quantité de tissus gras apparaissant comme de l'os. Cette ligne de base peut être modélisée et extrapolée dans les zones ne contenant pas d'os puis soustraite de l'image d'os.

Pour la détermination de la densité osseuse, la conicité induit un biais sur ces mesures, conduisant à une relation entre reproductibilité et position de la zone osseuse dans l'acquisition. Afin de limiter cette sensibilité, on corrige l'effet de conicité sur l'aire mesurée en utilisant les paramètres géométriques du système d'acquisition déterminés dans l'étape "hors ligne". La mesure de densité osseuse n'est, quant à elle, pas affectée (ou très faiblement affectée en raison de la discrétisation) par l'effet de conicité.

On explique maintenant le procédé de correction des défauts d'une image provenant d'un détecteur de rayons X (ou γ) de type matriciel.

Pour éviter toute confusion avec la présente invention, ce procédé est appelé "procédé PCD" dans ce qui suit.

Ce procédé PCD est un procédé de correction des défauts d'images X ou γ, fondé sur la construction d'une carte de confiance regroupant les indices de confiance (continus, compris entre 0 et 1) de tous les pixels du détecteur.

De façon plus précise, ce procédé PCD est un procédé de correction des défauts d'images (relatifs à des pixels en défaut ou à des pixels non-mesurés par le détecteur), issus d'un détecteur de rayons X ou γ de type matriciel, consistant en une étape de calibrage ou calibration du détecteur, puis en une étape de correction des pixels en défaut de l'image à corriger. Le procédé PCD se caractérise par le fait que l'étape de calibration consiste en les opérations suivantes :

a) - acquisition d'une image noire ;
b) - acquisition d'au moins une image d'un objet connu ;
c) - détermination d'au moins une image d'offset et de gain moyen ;
d) - acquisition d'au moins une image de l'objet à mesurer avec une atténuation uniforme lorsque le détecteur est de type à rayons X et une émission uniforme lorsqu'il est de type à rayons γ ;
e) - détermination d'une première moyenne et d'un premier écart-type pour toute l'image de l'objet à mesurer ;
f) - réalisation d'une première carte de confiance en fonction du niveau de gris des pixels de l'image ainsi que de l'écart-type et de la moyenne déterminés en e) ;

et en ce que l'étape de correction consiste en l'acquisition d'une image de l'objet à mesurer et en une correction de l'image de l'objet à partir de la carte de confiance et de la dernière image de l'objet acquise.

Selon une variante de l'invention, l'étape de calibration consiste, après l'opération f) de réalisation d'une première carte de confiance, en les opérations suivantes :

g) - sur l'image de l'objet connu, détermination d'un gradient de niveau de gris ;

h) - détermination d'une seconde moyenne et d'un second écart-type, puis d'une seconde carte de confiance ; et

i) - détermination d'une troisième carte de confiance par produit, pixel à pixel, de la première et de la seconde cartes de confiance.

Avantageusement, la première et la seconde cartes de confiance sont constituées de l'ensemble des indices de confiance attribués à chaque pixel de l'image, l'indice de confiance de chaque pixel étant déterminé en fonction du niveau de gris de ce pixel, ainsi que de l'écart-type et de la moyenne de toute l'image de l'objet à mesurer.

Selon un mode de réalisation préféré du procédé PCD, l'étape b) d'acquisition d'image d'un objet connu est réalisée avec une atténuation uniforme.

Selon une variante du procédé PCD, dans laquelle les défauts d'images résultent de l'absence de mesure sur au moins une zone de l'image, l'étape de calibration consiste, après l'opération f) ou l'opération i), à :

- agrandir l'image de l'objet à mesurer ;
- ajouter, à la place de la zone de l'image sans mesure, des pixels ayant des indices de confiance nuls ; et
- déterminer une quatrième carte de confiance, à partir de la première ou de la troisième carte de confiance, en tenant compte de ces indices de confiance nuls ;

et l'étape de correction consiste à effectuer une nouvelle acquisition d'image de l'objet à mesurer, à lui appliquer l'agrandissement et à corriger l'image de l'objet à partir de la quatrième carte de confiance et de la dernière image de l'objet acquise.

On donne maintenant une description détaillée de modes de réalisation particuliers du procédé PCD.

Ce procédé PCD a pour but de corriger les pixels en défaut ou les pixels manquants d'images provenant d'un détecteur de rayons X ou $\gamma$ de type matriciel constitué de pixels. Plus précisément, ce procédé consiste à construire une carte de confiance à partir d'indices de confiance continus, compris entre 0 et 1, et représentant le niveau de confiance attribué à chaque pixel du détecteur. Il consiste ensuite à utiliser cette carte de confiance pour corriger les défauts du détecteur matriciel en utilisant un filtrage, tel que le filtrage de KNUTSSON, décrit ultérieurement, ou un filtrage par équations aux dérivés partielles (EDP).

Selon le procédé PCD, la détection des pixels en défaut n'est pas binaire, mais continue, ce qui permet un traitement plus précis au niveau des frontières des zones de pixels en défaut.

Le procédé PCD comporte deux étapes principales, à savoir :

- une étape de calibration du détecteur qui consiste à effectuer l'acquisition d'une ou plusieurs images d'un objet de référence connu ; puis à partir de ces images, à détecter les pixels en défaut, puis à déterminer la carte de confiance regroupant l'ensemble des indices de confiance de tous les pixels de l'image ; et
- une étape de correction qui consiste, à partir de la carte de confiance, à corriger l'image de l'objet à mesurer au moyen d'un filtrage, tel que celui proposé par KNUTSSON.

D'une façon générale, la détection des pixels en défaut se fait grâce à l'acquisition d'une ou plusieurs images d'un objet de référence connu qui, de préférence, a une atténuation uniforme.

Dans le cas où il n'y a aucun pixel en défaut sur le détecteur, l'image obtenue est uniforme, au bruit près créé par la génération des photons X ou $\gamma$, la détection et la conversion de ces rayons X ou $\gamma$ en valeurs numériques.

Dans le cas où il y a des pixels en défaut sur le détecteur, la détection de ces pixels en défaut se fait à partir d'une analyse statistique de l'image de calibration obtenue et de l'image de gradient correspondante, car un pixel en défaut a soit une valeur aberrante, soit un fort gradient. Lorsque les pixels en défaut sont détectés, on attribue à chacun de ces pixels un indice de confiance qui varie de façon continue entre 0 et 1.

Le cas de détection de défauts définis par un écart à la moyenne d'une image uniforme et le cas de détection de défaut par un pixel à fort gradient pour une acquisition a priori uniforme peuvent être combinés ; la combinaison de ces deux critères se fait grâce au produit des deux cartes de confiance effectués chacune sur un critère différent.

Dans ce procédé PCD, on fait l'hypothèse justifiée que la distribution des pixels en défaut est stable dans le temps. Les défauts vont donc être visibles sur toutes les images acquises sur le dispositif. En effet, la correction de pixels en défaut est spécifique au détecteur et non à l'image observée. Elle comble les manques d'information en utilisant au mieux les informations fournies par les pixels voisins et en assurant une continuité de l'information.

Les images ainsi corrigées sont plus facilement exploitables visuellement par un opérateur dans un objectif, notamment de diagnostic.

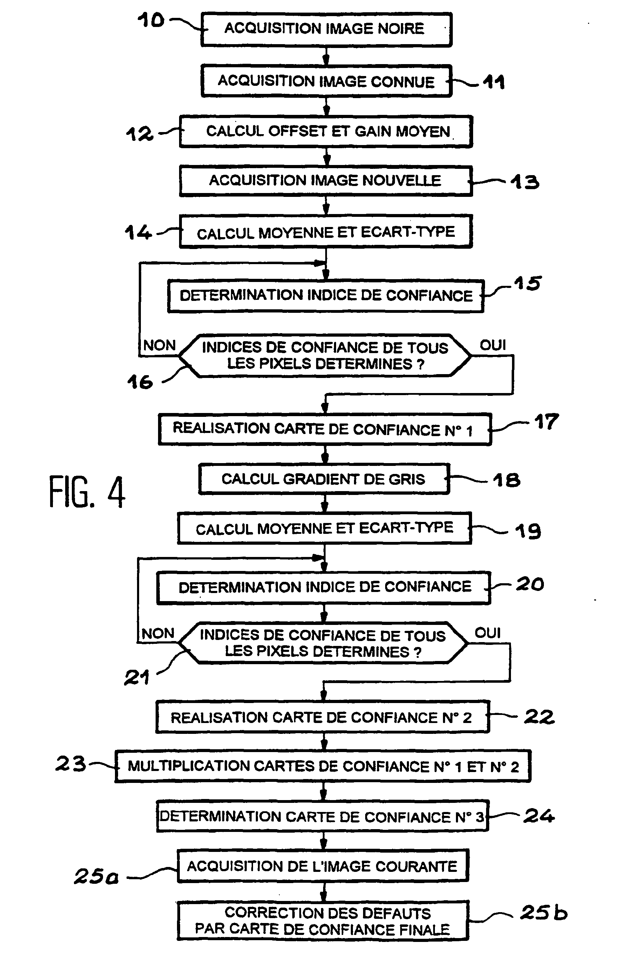

Sur la figure 4, on a représenté schématiquement le diagramme fonctionnel des étapes principales du procédé PCD. De façon plus précise, le procédé PCD comporte une étape 10 d'acquisition d'une ou de plusieurs images noires, c'est-à-dire des images effectuées dans le noir, sans émission de rayonnements X. Cette acquisition d'images noires permet de mesurer le bruit de l'électronique du dispositif de lecture associé au détecteur.

Le procédé PCD consiste ensuite en une étape 11 d'acquisition d'une ou de plusieurs images d'un objet de référence connu. Cette acquisition d'image de référence se fait, de préférence, avec une atténuation uniforme lorsqu'il s'agit d'un détecteur à rayons X.

L'étape suivante du procédé PCD, référencée 12, consiste à calculer des images d'offset et de gain moyens Moy_N et Moy_G, avec :

$$Moy\_N = 1/n \sum_{1}^{n} Nn$$

et

$$Moy\_G = 1/n \sum_{1}^{n} (Gn - Moy\_N)$$

où Gn représente les images de l'objet de référence connu, Nn représente les images noires, et où n est le nombre d'images acquises aussi bien dans l'étape 10 que dans l'étape 11.

Le procédé PCD se poursuit par une étape 13 qui consiste à effectuer l'acquisition d'une image Im de l'objet à mesurer, appelée « acquisition d'image nouvelle ». Cette acquisition s'effectue pour une atténuation uniforme, dans le cas d'un détecteur de rayons X. Cette image Im est alors corrigée de l'offset et du gain de la façon suivante :

$$Im\_cor(x, y) = K \cdot \left( \frac{Im - Moy\_N}{Moy\_G} \right)$$

où K est une constante choisie par l'utilisateur, et qui a uniquement pour but de définir la dynamique de niveau de gris pour coder la valeur des pixels de l'image corrigée.

Toutefois, la correction d'offset et de gain peut se faire selon des méthodes différentes de celle qui a été décrite précédemment et, par exemple, en utilisant une série d'objets d'atténuations différentes.

Une étape 14 consiste ensuite à déterminer l'écart-type (ou la variance) et la moyenne sur l'image corrigée Im_cor. Cette moyenne et cette variance sont calculées sur l'image corrigée de l'offset. La moyenne est déterminée par :

$$Moy = 1/_{nombre-pixel} \sum_{x} \sum_{y} Im\_cor(x, y)$$

et la variance par :

$$Var = 1/_{nombre\_pixel} \sum_{x} \sum_{y} (Im\_cor(x, y) - Moy)^2 \; .$$

Ces valeurs de moyenne et de variance peuvent éventuellement être affinées en supprimant l'effet des points aberrants. Pour cela, on recalcule la variance et la moyenne de l'image uniquement pour les pixels pour lesquels les différences de la valeur avec la moyenne Moy est inférieure d'un pourcentage x % à la racine carrée de la variance, x étant défini par l'opérateur.

Le procédé PCD se poursuit par une étape 15 qui consiste à déterminer l'indice de confiance de chaque pixel du détecteur. En d'autres termes, un indice de confiance compris de façon continue entre 0 et 1 est calculé pour chaque pixel de l'image en fonction de la valeur du niveau de gris du pixel, de la variance et de la moyenne déterminées pour l'image corrigée. Plus précisément, l'indice de confiance se détermine par :

- le niveau de gris du pixel, déterminé selon la normalisation des niveaux de gris de l'ensemble des pixels de façon à obtenir une distribution normale :

$$\text{norm}(x, y) = \left| \frac{\text{Im\_cor} - (x, y) - \text{Moy}}{\text{Var}} \right|$$

- la transformation de la valeur par la fonction F de répartition de la loi normale :

$$F(u) = \frac{1}{\sqrt{2\pi}} \int_{-\infty}^{u} e^{-y^2/2} dy$$

pour obtenir une distance dite « normale » :

$$\text{dist\_norm}(x, y) = F(\text{norm}(x, y))$$

- le calcul de l'indice de confiance du pixel :

$$C1(x, y) = 2(1 - \text{dist\_norm}(x, y)).$$

Pour un pixel dont le niveau de gris de départ est proche de la moyenne Moy, l'indice de confiance vaut 1 ; plus la valeur du niveau de gris du pixel s'éloigne de la valeur moyenne Moy, plus l'indice de confiance se rapproche de 0.

Le procédé PCD se poursuit par un test 16 qui consiste à vérifier si tous les indices de confiance de tous les pixels du détecteur ont bien été déterminés. Si ce n'est pas le cas, on continu la détermination des indices de confiance, à l'étape 15. Si c'est le cas, le procédé PCD se poursuit par l'étape 17 qui consiste à réaliser une première carte de confiance C1. Cette carte de confiance regroupe tous les indices de confiance déterminés dans l'étape 15.

Le procédé PCD se poursuit par une étape 18 qui consiste à déterminer, sur l'image initiale Im, déterminée à l'étape 13, le gradient de niveau de gris de l'image. Cette étape consiste, de façon plus précise :

- à calculer l'image de gradient grad_x(x, y) suivant la direction x de l'image corrigée en 13, par exemple par convolution de l'image Im_cor par un noyau $\begin{bmatrix} -1 & 0 & 1 \\ -2 & 0 & 2 \\ -1 & 0 & 1 \end{bmatrix}$ ;

- à calculer une image de gradient grad_y(x, y) selon la direction Y de l'image Im_cor, par exemple par convolution de l'image Im_cor par un noyau $\begin{bmatrix} -1 & -2 & -1 \\ 0 & 0 & 0 \\ 1 & 2 & 1 \end{bmatrix}$ ;

- à calculer le module du gradient à partir de l'expression :

$$Mod\_grad(x, y) = \sqrt{(grad\_x, y)^2 + (grad\_y(x, y)^2}.$$

Le procédé PCD se poursuit par une étape 19 de calcul de la variance et de la moyenne, de l'image de gradient obtenue à l'étape 18.

La variance et la moyenne de l'image de gradient se déterminent, respectivement, par :

$$Moy\_grad = 1 / nombre - pixel \sum_x \sum_y Mod\_grad(x, y)$$

$$Var\_grad = 1 / nombre\_pixel \sum_x \sum_y (Mod\_grad(x, y) - Moy\_grad)^2.$$

Ces valeurs de variance et de moyenne peuvent éventuellement être améliorées en supprimant l'effet des points aberrants, comme cela a déjà été expliqué pour le calcul de la première variance et de la première moyenne à l'étape 14.

Le procédé PCD se poursuit par une étape 20) qui consiste à déterminer l'indice de confiance de chaque pixel du détecteur, à partir de son niveau de gris et des moyenne et variance déterminées sur l'image de gradient. Autrement dit, l'indice de confiance de chaque pixel est déterminé à partir de :

- la normalisation des niveaux de gris de l'ensemble des pixels, afin d'obtenir une distribution normale :

$$norm\_grad(x, y) = \left| \frac{Mod\_grad(x, y) - Moy\_grad}{Var\_grad} \right|$$

- la transformation de la valeur par la fonction F de répartition de la loi normale $F(u) = \frac{1}{\sqrt{2\pi}} \int_{-\infty}^{u} e^{-y^2/2} \, dy$ pour obtenir une « distance normale » :

$$dist\_norm\_grad(x, y) = F(norm\_grad(x, y))$$

- le calcul de l'indice de confiance du pixel par :

$$C2(x, y) = 2(1 - dist\_norm\_grad(x, y)).$$

Le procédé PCD comporte alors un test 21 qui consiste à vérifier que l'indice de confiance de chaque pixel du détecteur a bien été déterminé. Tant que ce n'est pas le cas, l'étape 20 est réinitialisée pour chacun des pixels. Lorsque c'est le cas, le procédé PCD se poursuit par une étape 22 qui consiste à réaliser une seconde carte de confiance C2 qui est constituée de l'ensemble de tous les indices de confiance de tous les pixels du détecteur déterminés à l'étape 20.

Le procédé PCD se poursuit ensuite par une étape 23 qui consiste en une multiplication de la première et de la seconde cartes de confiance, de façon à obtenir une troisième carte de confiance C3 (étape 24). Ce sont les indices de confiance de cette troisième carte qui sont utilisés pour corriger les pixels en défaut de l'image de l'objet à mesurer (étape 25) ; en effet, chaque pixel en défaut est remplacé par l'indice de confiance qui lui correspond sur la carte de confiance C3. La détermination de cette troisième carte de confiance, appelée carte de confiance finale, est déterminée à partir de l'équation :

$$C\_final(x, y) = C1(x, y).C2(x, y).$$

La calibration du détecteur est effectuée une seule fois. Ensuite, on peut effectuer l'acquisition de plusieurs images sur le détecteur et les corriger, sans avoir à recalibrer le détecteur.

L'étape 25 de correction consiste d'abord à effectuer l'acquisition d'une image de l'objet (25a) appelée image courante. L'étape 25b de correction de l'image peut être réalisée au moyen d'un filtrage, tel que celui proposé par KNUTSSON, en utilisant la carte de confiance finale calculée dans l'étape 24 de calibration. Ce filtrage est décrit, notamment, dans le document de H. KNUTSSON et C. WESTING, « Normalized and Differential Convolution, Method for Interpolation and Filtering of Incomplete and Uncertain Data », Proceedings CVPR 93, pp. 515-523, New York 1993.

Dans le cas le plus simple (à l'ordre 0), la méthode peut être résumée de façon suivante :

- on considère une image I qui, par exemple, est l'image acquise sur le détecteur X ;
- par ailleurs, on considère que l'on dispose d'une image de confiance C sur les pixels de l'image ;
- on peut alors construire une nouvelle image filt_I de la façon suivante :

$$filt\_I = (I.C)*a/C*a$$

où :

- « . » et « / » sont les opérateurs de multiplication et division d'image pixel à pixel ;
- « * » est l'opérateur de convolution ;
- a est le noyau de convolution suivant :

$$a = \begin{cases} r^{-\alpha} \cos^{\beta}\left(\dfrac{\pi r}{2 r_{max}}\right) \text{ pour } r < r_{max} \\ \quad 0 \quad \text{ pour } r \geq r_{max} \end{cases}$$

L'image filt_I ainsi obtenue est une image pour laquelle :

- sur les zones qui ont un indice de confiance uniforme, l'effet du traitement est équivalent à un filtrage ;
- pour les pixels dont la confiance est inférieure à ses voisins, l'effet du traitement est de type « interpolation douce » avec une nouvelle valeur qui dépendra en majorité des pixels voisins de forte confiance.

La taille et la forme du noyau de convolution est à adapter à la taille (diamètre minimum) des défauts que l'on veut corriger.

Le filtrage, selon la méthode de KNUTSSON, permet de corriger les défauts d'image en utilisant au mieux l'information fournie par les pixels corrects de l'image. Par ailleurs, dans le procédé PCD, afin d'éviter d'introduire un effet de filtrage, même sur les zones correctes où la confiance sur les pixels est bonne, le procédé PCD propose de combiner l'image initiale et l'image corrigée de façon à créer une nouvelle image corrigée pondérée par les indices de confiance. Cela se traduit par l'équation :

$$\texttt{Corr\_I = Im.C\_final + (1 - C\_final).filt\_I,}$$

où filt_I est l'image filtrée par la méthode de KNUTSSON.

La correction des pixels en défaut à partir de la troisième carte de confiance peut être réalisée par d'autres méthodes de filtrage, comme la méthode de filtrage par équations aux dérivés partielles, décrite dans le document de R. DE RICHES et O. FAUGERAS, intitulé « Les équations aux dérivés partielles en traitement des images et vision par ordinateur », publié dans la revue « Traitement du signal », volume 13, n° 6, pages 552 à 577, Février 1996.

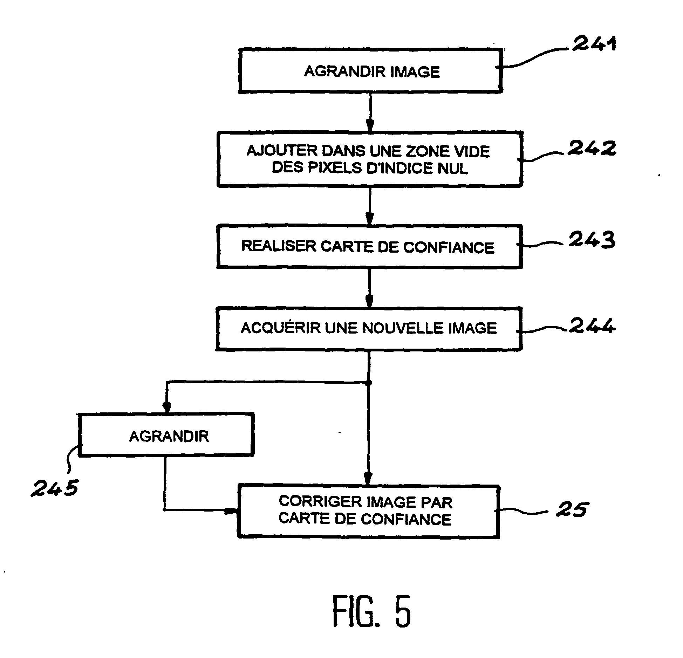

Une variante du procédé PCD concerne le cas où les défauts de l'image sont dus à l'absence de mesure sur une ou plusieurs zones du détecteur. Dans ce cas, le procédé PCD consiste à effectuer des étapes supplémentaires après l'étape 24 de détermination de la troisième carte de confiance. Ces étapes sont représentées sur la figure 5.

Dans cette variante, le procédé PCD comporte une étape 241 qui consiste à agrandir l'image de l'objet de façon à introduire les zones de pixels sans mesure (ou pixels manquants) où le détecteur n'a pas pu faire de mesure. En effet, l'image acquise est de plus petite taille que l'image qui aurait été acquise avec un détecteur apte à détecter sur toute sa surface. Une étape 242 consiste ensuite à ajouter, dans ces zones, des pixels d'indices de confiance nuls. Une étape 243 consiste ensuite à réaliser une quatrième carte de confiance qui prend en compte les indices de confiance nuls des pixels de la zone sans mesure. Cette quatrième carte de confiance est alors considérée comme la carte finale.

Le détecteur étant calibré, une étape 244 consiste ensuite à effectuer une nouvelle acquisition d'image de l'objet à mesurer, ou image courante. Cette dernière est agrandie (étape 245) puis corrigée. C'est à partir de cette image courante agrandie, et en appliquant la quatrième carte de confiance, que l'image est corrigée, dans l'étape 25 décrite précédemment.

En d'autres termes, cette variante consiste à introduire les zones sans information, c'est-à-dire les pixels pour lesquels le détecteur n'a pas pu faire de mesure, dans l'image de confiance finale établie à l'étape 24 ; cela se fait en augmentant la taille de l'image de confiance. Pour cela, on coupe l'image au niveau des zones manquantes et on ajoute des pixels à indices de confiance nuls pour remplir les vides de l'image.

**Revendications**

1. Procédé d'utilisation d'un système (1) d'ostéodensitométrie par rayons X à au moins deux énergies, à faisceau conique, ce système comprenant une source (1a) de rayons X apte à fournir un faisceau conique de rayons X à au moins une première énergie, appelée haute énergie, et une deuxième énergie, appelée basse énergie et inférieure à la première énergie, un détecteur bidimensionnel (2) de rayons X et des moyens électroniques (3a, 3b) de traitement des images fournies par ce détecteur, ce procédé étant **caractérisé en ce qu'**on acquiert l'image à basse énergie d'une zone anatomique d'un patient (1c), et pour prendre en compte les mouvements du patient dans les mesures de densité osseuse, on acquiert l'image à haute énergie de cette zone anatomique et l'on met en correspondance ces images respectivement acquises à basse énergie et à haute énergie, avant de construire la carte des densités osseuses de la zone anatomique.

2. Procédé selon la revendication 1, dans lequel, pour mettre en correspondance les images respectivement acquises à haute et basse énergies :

   - on extrait respectivement de ces images les ensembles de contours des zones osseuses de la zone anatomique,
   - on recherche la transformation plane optimale permettant de mettre l'ensemble de contours relatif à l'image acquise à haute énergie en correspondance avec l'ensemble de contours relatif à l'image acquise à basse énergie,
   - on utilise cette transformation pour amener l'image acquise à haute énergie dans le référentiel de l'image acquise à basse énergie, et
   - on combine ces deux images pour déterminer la carte de mesure des densités osseuses.

**3.** Procédé selon la revendication 1, dans lequel, pour mettre en correspondance les images respectivement acquises à haute et basse énergies :

- on extrait respectivement de ces images les ensembles de contours des zones osseuses de la zone anatomique,
- on recherche la transformation plane optimale permettant de mettre l'ensemble de contours relatif à l'image acquise à basse énergie en correspondance avec l'ensemble de contours relatif à l'image acquise à haute énergie,
- on utilise cette transformation pour amener l'image acquise à basse énergie dans le référentiel de l'image acquise à haute énergie, et
- on combine ces deux images pour déterminer la carte de mesure des densités osseuses.

**4.** Procédé selon l'une quelconque des revendications 1 à 3, dans lequel, avant de faire les acquisitions à haute et basse énergies, on fait un cliché, à faible dose de rayons X, de la zone anatomique du patient (1c) pour aider à positionner cette zone dans le système.

**5.** Procédé selon la revendication 4, dans lequel, lorsque le patient (1c) est soumis à un premier examen, on utilise le cliché à faible dose de rayons X pour rétroagir sur la mécanique du système (1) afin de positionner la zone anatomique par rapport à un référentiel préétabli.

**6.** Procédé selon la revendication 4, dans lequel lorsque le patient (1c) est soumis à un examen de suivi, on utilise le cliché à faible dose de rayons X pour placer la zone anatomique dans une position identique à celle qu'elle occupait lors de l'examen précédent.

**7.** Procédé selon l'une quelconque des revendications 1 à 3, dans lequel, avant de faire les acquisitions à haute et basse énergies, on fait un cliché à faible dose de rayons X, pour adapter la dose d'irradiation par réglage du flux de rayons X en modifiant le courant appliqué à la source de rayons X utilisée et/ou la tension appliquée à cette source.

**8.** Procédé selon l'une quelconque des revendications 1 à 3, dans lequel, avant de faire les acquisitions à haute et basse énergies, on fait un cliché à faible dose de rayons X pour positionner automatiquement des caches permettant de limiter la zone d'irradiation.

**9.** Système (1) d'ostéodensitométrie par rayons X à au moins deux énergies, à faisceau conique, ce système comprenant une source (1a) de rayons X apte à fournir un faisceau conique de rayons X à au moins une première énergie, appelée haute énergie, et une deuxième énergie, appelée basse énergie et inférieure à la première énergie, un détecteur bidimensionnel (2) de rayons X et des moyens électroniques (3a, 3b) de traitement des images fournies par ce détecteur, **caractérisé en ce que** les moyens électroniques de traitement sont prévus pour mettre en correspondance une image acquise à basse énergie d'une zone anatomique d'un patient et une image acquise à haute énergie de cette zone anatomique, avant de construire la carte des densités osseuses de la zone anatomique, afin de prendre en compte les mouvements du patient dans les mesures de densité osseuse.

### Claims

**1.** Process for use of an osteodensitometry system (1) using cone beam X-rays with at least two energies, this system comprising an X-ray source (1a) capable of supplying a cone X-ray beam with at least a first energy called the high energy and a second energy called the low energy and less than the first energy, a two dimensional X-ray detector (2) and electronic means (3a, 3b) for processing images supplied by this detector, this process being **characterised in that** the low energy image of a part of the anatomy of a patient (1c) is acquired, and the high energy image of this part of the anatomy is acquired to take patient movements into account in the bone density measurements, and these images acquired at low energy and at high energy are matched before building up the map of bone densities of the part of the anatomy.

**2.** Process according to claim 1, in which the following procedure is used to match the images acquired at high and low energies respectively:

- sets of contours of bone areas in the part of the anatomy are extracted from these images,
- a search is made for an optimum plane transformation in order to match the set of contours for the image acquired at high energy with a set of contours for the image acquired at low energy,
- this transformation is used to bring the image acquired at high energy into the coordinate system of the image acquired at low energy, and
- these two images are combined to determine the bone density measurement map.

3. Process according to claim 1, in which the following process is used to match the images acquired at high and low energies respectively:

- sets of contours of bone areas in the part of the anatomy are extracted from these images,
- a search is made for an optimum plane transformation in order to match the set of contours for the image acquired at low energy with a set of contours for the image acquired at high energy,
- this transformation is used to bring the image acquired at low energy into the coordinate system of the image acquired at high energy, and
- these two images are combined to determine the bone density measurement map.

4. Process according to any one of claims 1 to 3, in which a plate with a low X-ray dose is made of the part of the anatomy of the patient (1c), to help in positioning this area in the system, before making the acquisitions at high and low energies.

5. Process according to claim 4 in which, when the patient (1c) is examined for the first time, this low X-ray dose plate is used to retroact on the mechanics of the system (1) in order to position the part of the anatomy with respect to the predetermined coordinate system.

6. Process according to claim 4 in which, when the patient (1c) is subjected to a check-up examination, the plate taken at a low X-ray dose will be used to put the part of the anatomy into exactly the same position that it was in during the previous examination.

7. Process according to any one of claims 1 to 3 in which a plate with a low X-ray dose is made before making acquisitions at low and high energies, in order to adapt the irradiation dose by adjusting the X-ray flux by modifying the current applied to the X-ray source used and / or the voltage applied to this source.

8. Process according to any one of claims 1 to 3 in which a plate with a low X-ray dose is made before making the high and low energy acquisitions, in order to automatically position masks in order to limit the irradiation area.

9. Osteodensitometry system (1) using cone beam X-rays with at least two energies, this system comprising an X-ray source (1a) capable of supplying a cone X-ray beam with at least a first energy called the high energy and a second energy called the low energy and less than the first energy, a two dimensional X-ray detector (2) and electronic means (3a, 3b) for processing images supplied by this detector, **characterised in that** the electronic processing means are designed to match an image of part of the anatomy of the patient acquired at low energy and an image of this part of the anatomy acquired at high energy, before building up the bone density map of the part of the anatomy, in order to take patient movements into account in the bone density measurements.

**Patentansprüche**

1. Verfahren zur Anwendung eines Knochendichtemessungssystems (1) mittels Röntgenstrahlung bei wenigstens zwei Energien, mit konischem Strahl, wobei dieses System eine Röntgenstrahlungsquelle (1a), fähig einen konischen Röntgenstrahl mit wenigstens einer ersten Energie, Hochenergie genannt, und einer zweiten Energie, Niederenergie genannt und niedriger als die erste Energie, einen zweidimensionalen Röntgenstrahlungsdetektor (2) und elektronische Einrichtungen (3a, 3b) zur Verarbeitung der durch diesen Detektor gelieferten Bilder umfasst, **dadurch gekennzeichnet, dass** man das Niederenergie-Bild einer anatomischen Zone eines Patienten (1c) erfasst und, um den Bewegungen des Patienten bei den Knochendichtemessungen Rechnung zu tragen, das Hochenergie-Bild dieser anatomischen Zone erfasst, und man diese jeweils bei Niederenergie und bei Hochenergie erfassten Bilder in Übereinstimmung bringt, ehe man die Karte der Knochendichten der anatomischen Zone konstruiert.

2. Verfahren nach Anspruch 1, bei dem - um die jeweils bei Hoch- und Niederenergie erfassten Bilder in Übereinstimmung zu bringen:

- man diesen Bildern jeweils die Gesamtheit der Konturen der Knochenzonen der anatomischen Zone entnimmt,
- man die optimale plane Transformation sucht, die ermöglicht, die Gesamtheit der das mit Hochenergie erfasste Bild betreffenden Konturen in Übereinstimmung zu bringen mit der Gesamtheit der das mit Niederenergie erfasste Bild betreffenden Konturen,
- man diese Transformation benutzt, um das mit Hochenergie erfasste Bild in das Bezugssystem des bei Niederenergie erfassten Bildes zu bringen, und
- man diese beiden Bilder kombiniert, um die Mess- bzw. Maßkarte der Knochendichten zu bestimmen.

3. Verfahren nach Anspruch 1, bei dem - um die jeweils bei Hoch- und Niederenergie erfassten Bilder in Übereinstimmung zu bringen:

- man diesen Bildern jeweils die Gesamtheit der Konturen der Knochenzonen der anatomischen Zone entnimmt,
- man die optimale plane Transformation sucht, die ermöglicht, die Gesamtheit der das mit Niederenergie erfasste Bild betreffenden Konturen in Übereinstimmung zu bringen mit der Gesamtheit der das mit Hochenergie erfasste Bild betreffenden Konturen,
- man diese Transformation benutzt, um das mit Hochenergie erfasste Bild in das Bezugssystem des bei Niederenergie erfassten Bildes zu bringen, und
- man diese beiden Bilder kombiniert, um die Mess- bzw. Maßkarte der Knochendichten zu bestimmen.

4. Verfahren nach einem der Ansprüche 1 bis 3, bei dem man, ehe man die Erfassungen bei Hoch- und Niederenergie durchführt, eine Aufnahme der anatomischen Zone des Patienten (1c) mit einer niedrigen Röntgenstrahlungsdosis macht, um zu helfen, diese Zone in dem System zu positionieren.

5. Verfahren nach Anspruch 4, bei dem man, wenn der Patient (1c) einer ersten Untersuchung unterzogen wird, die Aufnahme mit der niedrigen Röntgenstrahlungsdosis benutzt, um rückzuwirken auf den Mechanismus des Systems (1), um die anatomische Zone in Bezug auf ein im Voraus festgelegtes Bezugssystem zu positionieren.

6. Verfahren nach Anspruch 4, bei dem man, wenn der Patient (1 c) einer ersten Untersuchung unterzogen wird, die Aufnahme mit der niedrigen Röntgenstrahlungsdosis benutzt, um die anatomische Zone in einer Position zu platzieren, die mit derjenigen identisch ist, die sie während der vorhergehenden Untersuchung eingenommen hat.

7. Verfahren nach einem der Ansprüche 1 bis 3, bei dem man, ehe man die Erfassungen bei Hoch- und Niederenergie durchführt, eine Aufnahme der anatomischen Zone mit einer niedrigen Röntgenstrahlungsdosis macht, um die Strahlungsdosis durch Regelung des Röntgenstrahlenflusses anzupassen, indem man den in die benutzte Röntgenstrahlungsquelle eingespeisten Strom und/oder die an diese Quelle angelegte Spannung modifiziert.

8. Verfahren nach einem der Ansprüche 1 bis 3, bei dem man, ehe man die Erfassungen bei Hoch- und Niederenergie durchführt, eine Aufnahme der anatomischen Zone mit einer niedrigen Röntgenstrahlungsdosis macht, um automatisch Abdeckungen zu positionieren, die ermöglichen, die Bestrahlungszone zu begrenzen.

9. System (1) zur Knochendichtemessung mittels Röntgenstrahlen bei wenigstens zwei Energien, mit konischem Strahl, wobei dieses System eine Röntgenstrahlenquelle (1a), fähig einen konischen Röntgenstrahl mit wenigstens einer Energie, Hochenergie genannt, und einer zweiten Energie, Niederenergie genannt und niedriger als die erste Energie, einen zweidimensionalen Röntgenstrahlendetektor (2) und elektronische Einrichtungen (3a, 3b) zur Verarbeitung der durch diesen Detektor gelieferten Bilder umfasst, **dadurch gekennzeichnet, dass** die elektronischen Verarbeitungseinrichtungen vorgesehen sind, ein bei Niederenergie von einer anatomischen Zone eines Patienten erfasstes Bild und ein bei Hochenergie von dieser anatomischen Zone erfasstes Bild vor der Konstruktion der Karte der Knochendichten der anatomischen Zone in Übereinstimmung zu bringen, um den Bewegungen des Patienten bei den Knochendichtemessungen Rechnung zu tragen.

FIG. 1

FIG. 2

**F1** — POSITIONNEMENT GROSSIER DU PATIENT POUR OBSERVER LA ZONE ANATOMIQUE D'INTÉRÊT

**F2** — EXTRACTION DES CONTOURS DE LA STRUCTURE OSSEUSE

**F3** — IDENTIFICATION DE POINTS CARACTÉRISTIQUES

OUI — EST-CE LE PREMIER EXAMEN ? — NON

**F4**

**F5**

IDENTIFICATION DE LA TRANSFORMATION GÉOMÉTRIQUE AMENANT LES POINTS CARACTÉRISTIQUES EN POSITION STANDARD

**F7**

RÉCUPÉRATION DE LA POSITION DES POINTS CARACTÉRISTIQUES DANS L'EXAMEN ANTÉRIEUR

APPLICATION D'UN MOUVEMENT À LA MÉCANIQUE POUR AMENER CES POINTS VERS LA POSITION STANDARD

**F6**

IDENTIFICATION DE LA TRANSFORMATION GÉOMÉTRIQUE AMENANT LES POINTS CARACTÉRISTIQUES COURANTS VERS CEUX DE L'EXAMEN ANTÉRIEUR

**F8**

**FIG. 3**

APPLICATION D'UN MOUVEMENT À LA MÉCANIQUE POUR AMENER LE PATIENT VERS UNE POSITION CORRESPONDANT À UN BON PLACEMENT DES POINTS CARACTÉRISTIQUES

**F9**

10 — ACQUISITION IMAGE NOIRE

ACQUISITION IMAGE CONNUE — 11

12 — CALCUL OFFSET ET GAIN MOYEN

ACQUISITION IMAGE NOUVELLE — 13

14 — CALCUL MOYENNE ET ECART-TYPE

DETERMINATION INDICE DE CONFIANCE — 15

NON / INDICES DE CONFIANCE DE TOUS LES PIXELS DETERMINES ? \ OUI
16

REALISATION CARTE DE CONFIANCE N° 1 — 17

**FIG. 4**

CALCUL GRADIENT DE GRIS — 18

CALCUL MOYENNE ET ECART-TYPE — 19

DETERMINATION INDICE DE CONFIANCE — 20

NON / INDICES DE CONFIANCE DE TOUS LES PIXELS DETERMINES ? \ OUI
21

REALISATION CARTE DE CONFIANCE N° 2 — 22

23 — MULTIPLICATION CARTES DE CONFIANCE N° 1 ET N° 2

DETERMINATION CARTE DE CONFIANCE N° 3 — 24

25a — ACQUISITION DE L'IMAGE COURANTE

CORRECTION DES DEFAUTS PAR CARTE DE CONFIANCE FINALE — 25b

FIG. 5